(19) Europäisches Patentamt
European Patent Office
Office européen des brevets

(11) **EP 3 112 870 B1**

(12) **EUROPEAN PATENT SPECIFICATION**

(45) Date of publication and mention
of the grant of the patent:
**27.05.2020 Bulletin 2020/22**

(21) Application number: **15754701.9**

(22) Date of filing: **26.01.2015**

(51) Int Cl.:
***G01N 33/543*** *(2006.01)*  ***G01N 21/64*** *(2006.01)*

(86) International application number:
**PCT/JP2015/052054**

(87) International publication number:
**WO 2015/129361 (03.09.2015 Gazette 2015/35)**

(54) **SENSOR CHIP FOR SURFACE PLASMON-FIELD ENHANCED FLUORESCENCE SPECTROSCOPY COMPRISING DIFFERENT BLOCKING AGENTS**

SENSORCHIP FÜR OBERFLÄCHENPLASMONEN-FELDVERSTÄRKTE FLUORESZENZSPEKTROSKOPIE MIT VERSCHIEDENEN BLOCKIERUNGSMITTELN

PUCE DE DÉTECTION POUR SPECTROSCOPIE DE FLUORESCENCE ACTIVÉE PAR CHAMP DE PLASMONS DE SURFACE AVEC DES AGENTS DE BLOCAGE DIFFÉRENTS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.02.2014 JP 2014035177**

(43) Date of publication of application:
**04.01.2017 Bulletin 2017/01**

(73) Proprietor: **Konica Minolta, Inc.**
**Tokyo 100-7015 (JP)**

(72) Inventors:
• **HIKAGE, Naoki**
**Tokyo 100-7015 (JP)**
• **HIRAYAMA, Hiroshi**
**Tokyo 100-7015 (JP)**
• **WADA, Takeshi**
**Tokyo 100-7015 (JP)**
• **AOKI, Youichi**
**Tokyo 100-7015 (JP)**

(74) Representative: **Gille Hrabal**
**Brucknerstrasse 20**
**40593 Düsseldorf (DE)**

(56) References cited:
EP-A1- 2 306 176     WO-A1-2007/145180
WO-A1-2009/054091     WO-A1-2009/091023
WO-A1-2012/023391     WO-A2-2009/021964
JP-A- 2002 040 026     JP-A- 2006 308 321
JP-A- 2006 322 854     JP-A- 2013 178 224
US-A1- 2008 014 575     US-A1- 2009 079 978

**Description**

TECHNICAL FIELD

The present invention relates to a sensor chip for surface plasmon-field enhanced fluorescence spectroscopy, in which non-specific adsorption of contaminants originating from a measurement sample (biological sample) to a sensor section is inhibited. More particularly, the present invention relates to a sensor chip for surface plasmon-field enhanced fluorescence spectroscopy, in which non-specific adsorption of contaminants originating from a measurement sample (biological sample) to a sensor section is inhibited and generation of noise caused by autofluorescence generated from a dielectric member is reduced.

BACKGROUND ART

Detection and quantification of a tumor marker, a specific protein or nucleic acid or other biologically relevant substance contained in blood or urine of human or animal or in other biological sample are widely performed for diagnosis in today's medical field as well as for research in the fields of biology and biochemistry. As a method for measuring a trace amount of a biologically relevant substance contained in a biological sample with high sensitivity, a measurement method using surface plasmon-field enhanced fluorescence spectroscopy (hereinafter, also referred to as "SPFS") is known.

In SPFS, under a condition where attenuated total reflection (ATR) of an excitation light such as a laser beam irradiated from a light source occurs at the surface of a metal thin film, the surface of the metal thin film is allowed to generate surface plasmon (compression wave). As a result, the amount of photons included in the excitation light irradiated from the light source is increased by several ten times to several hundred times and an electric field-enhancing effect of the surface plasmon light is thus obtained. Further, in a measurement method using SPFS, by utilizing this electric field-enhancing effect to efficiently excite a fluorescent substance bound with a compound to be measured that is captured in the vicinity of the surface of the metal thin film and observing the thus generated fluorescence, even an infinitesimal amount of the compound to be measured can be detected.

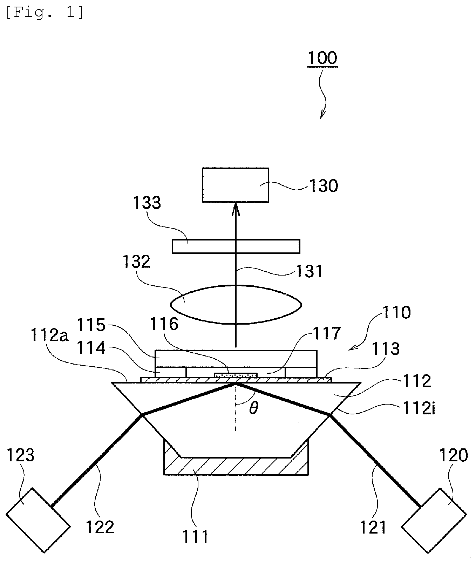

Fig. 1 shows one example of the schematic structure of a surface plasmon-field enhanced fluorescence spectroscopy apparatus (hereinafter, also referred to as "SPFS apparatus"). An SPFS apparatus 100 comprises a sensor chip mounting section 111 and is configured such that a sensor chip 110 is mounted on this sensor chip mounting section 111.

In the example shown in Fig. 1, the sensor chip 110 comprises: a dielectric member 112; a metal thin film 113, which is formed on a main surface 112a of the dielectric member 112; and a sensor section 116, which is formed at a prescribed position of a fine flow channel 117 on the metal thin film 113. The sensor section 116 is a region where a substance that captures a substance to be measured (hereinafter, also referred to as "capturing substance") is immobilized. The fine flow channel 117 is formed by a thin-layer member 114 and a plate (cover) 115 on the main surface 112a of the dielectric member 112 via the metal thin film 113.

The SPFS apparatus 100 also comprises, on the side of the dielectric member 112 of the sensor chip 110 mounted on the sensor chip mounting section 111: a light source 120, which irradiates an excitation light 121 that enters an incident surface 112i of the dielectric member 112 and travels toward the sensor section 116 at a prescribed incident angle $\theta$ that causes attenuated total reflection (ATR) on the metal thin film 113; and a light-receiving means 123, which receives a reflected light 122 that is irradiated from the light source 120 as the excitation light 121 and reflected by the metal thin film 113. Further, above the sensor chip 110, a light-detecting means 130, which receives fluorescence 131 emitted by a fluorescent substance labeling the substance to be measured that is captured on the sensor section 116, is arranged.

Between the sensor chip 110 and the light-detecting means 130, a light-condensing member 132 for efficiently condensing the fluorescence 131 and a wavelength-selecting function member 133, which removes light other than the fluorescence 131 and selectively allows only the fluorescence 131 to pass therethrough, are arranged.

Such SPFS apparatus 100 is used, for example, as follows. First, a sample liquid (measurement sample) containing the substance to be measured is introduced to the sensor section 116 via the fine flow channel 117 so as to allow the capturing substance immobilized on the sensor section 116 to capture the substance to be measured. Next, a substance (e.g., fluorescently labeled secondary antibody) that fluorescently labels the substance to be measured (hereinafter, such a substance is also referred to as "fluorescent labeling substance") is introduced in the same manner via the fine flow channel 117 so as to create a condition where the substance to be measured that is labeled with a fluorescent substance is captured on the sensor section 116.

Then, under this condition, by irradiating the excitation light 121 from the light source 120 via the dielectric member 112 at a prescribed incident angle $\theta$ that causes attenuated total reflection on the metal thin film 113, an enhanced electric field is generated due to resonance between evanescent wave and surface plasmon produced by the metal thin film 113, whereby the fluorescence 131 emitted by the fluorescent substance labeling the substance to be measured that is captured on the sensor section 116 is efficiently excited. By detecting the thus excited fluorescence 131 using the light-detecting means 130, even an extremely small amount of the substance to be measured can be detected and quantified.

In cases where a substance to be measured in a biological sample is measured using such an SPFS apparatus, noise is also generated in addition to the fluorescence 131 emitted by the fluorescent substance labeling the substance to be measured that is captured on the sensor section 116. This noise is mainly composed of noise (blank signal) that is generated by non-specific binding of the fluorescent labeling substance to the sensor section 116; and noise (baseline signal) that is generated by autofluorescence from the base materials of the dielectric member 112 and the like as well as stray light originating from the environment.

Particularly, the main causes of the blank signal include: non-specific adsorption of proteins, lipids, saccharides or other contaminants, which are contained in a biological sample in addition to the substance to be measured, to the sensor section (for example, the capturing substance (e.g., primary antibody) in the sensor section or a support used for immobilization of the capturing substance) and subsequent binding of a fluorescent labeling substance to these contaminants; and direct and non-specific adsorption of a fluorescent labeling substance to the sensor section. As a method for inhibiting such non-specific adsorption of contaminants and fluorescently labeling substances, a treatment with a blocking agent (hereinafter, referred to as "blocking treatment") is known (Patent Document 1).

Further, one of the causes of the baseline signal is autofluorescence generated by the material of the dielectric member such as a prism; however, since such autofluorescence is mostly blocked by the metal thin film, it does not reach the light-detecting means and the baseline signal is thus suppressed.

Conventionally, in a sensor chip comprising a fine flow channel, a blocking treatment is performed by introducing a blocking agent-containing solution to the fine flow channel and covering the entirety of a metal thin film in the fine flow channel with the blocking agent-containing solution. By this blocking treatment, non-specific adsorption of contaminants to a sensor section is inhibited. However, there are cases where the metal thin film is partially detached from a dielectric member with lapse of time. That is, the metal thin film is usually formed on a main surface of the dielectric member by a plasma-assisted sputtering method, a vacuum film-forming method such as electron beam-heating vacuum vapor deposition, or the like; however, after the metal thin film is subjected to a blocking treatment as described above, a phenomenon that the metal thin film partially swells in a circular form and is detached from the dielectric member occurs with time in some cases. It is now understood that, as a result of such phenomenon, the autofluorescence generated by the material of the dielectric member is no longer sufficiently blocked by the metal thin film, and this leads to an increase in the baseline signal and generation of noise.

Document US2009/079978 discloses a surface plasmon resonance sensor with binding areas which capture a specific substance to be analysed, and a blocking agent formed on the binding areas.

PRIOR ART REFERENCE

PATENT DOCUMENT

[Patent Document 1] JP-A-2006-292472

SUMMARY OF THE INVENTION

PROBLEMS TO BE SOLVED BY THE INVENTION

An object of the present invention is to provide a sensor chip according to claim 1 in which non-specific adsorption of contaminants originating from a measurement sample (biological sample) to a sensor section is effectively inhibited and the effect of blocking autofluorescence emitted by a dielectric member is not reduced with time.

TECHNICAL SOLUTION

The present inventors focused on that reduction with time in the effect of blocking autofluorescence emitted by the dielectric member is caused by partial detachment of the metal thin film from the dielectric member. As a result of investigation, the present inventors discovered that the detachment of the metal thin film from the dielectric member is affected by a blocking agent existing on the metal thin film; and that, as a cause thereof, when a blocking agent solution is fed to a fine flow channel formed as described in Patent Document 1 and a blocking treatment is performed on the entire inner wall of the fine flow channel that includes the metal thin film surface corresponding to the bottom surface thereof, the blocking agent solution remains in the fine flow channel (on the metal thin film surface) and the moisture thereof potentially causes the swelling and detachment of the metal thin film. The present inventors further discovered that, for inhibition of detachment of the metal thin film from the dielectric member, it is effective to restrict the range of blocking treatment only to a necessary area that includes the sensor section, thereby completing the present invention.

Moreover, the present inventors also discovered that, in the case of a sensor chip in which different kinds of capturing substances are each immobilized in different regions, in order to realize both inhibition of non-specific adsorption of contaminants and prevention of reduction in the effect of blocking autofluorescence emitted by the dielectric member, it is effective to perform blocking treatment only in necessary areas that each include a sensor section using blocking agents suitable for the substances captured by the capturing substances immobilized in the respective sensor sections, that is, blocking agents suitable for the substances to be measured that are captured in the respective sensor sections, thereby completing the present invention.

That is, the sensor chip for surface plasmon-field enhanced spectroscopy according to the present invention is as follows.

[1] A sensor chip for surface plasmon-field enhanced spectroscopy, comprising: a dielectric member; a metal thin film formed on a main surface of the dielectric member; and a region on a part of the metal thin film, where a capturing substance that specifically captures a substance to be measured is immobilized, wherein a blocking treatment with a blocking agent is performed in a region that includes the region where the capturing substance is immobilized, and the blocking treatment is not performed over the entirety of a wetted surface between a measurement sample and the metal thin film.

[2] The sensor chip according to [1], wherein the blocking treatment is performed only in the region where the capturing substance is immobilized.

[3] The sensor chip according to [1] or [2], wherein the sensor chip comprises plural regions where the capturing substance is immobilized, and at least one of blocking agents used for the blocking treatment performed in each of the plural regions is different from other blocking agent(s).

[4] The sensor chip according to any one of [1] to [3], wherein the blocking agent contains a substance having a blocking effect and a saccharide.

[5] The sensor chip according to any one of [1] to [4], wherein the blocking agent is selected from the group comprising bovine serum albumin, casein, gelatin and skim milk.

ADVANTAGEOUS EFFECTS OF INVENTION

According to the present invention, a sensor chip in which non-specific adsorption of contaminants originating from a measurement sample (biological sample) to a sensor section is effectively inhibited and the effect of blocking autofluorescence emitted by a dielectric member is not reduced with time is provided.

BRIEF DESCRIPTION OF DRAWINGS

[Fig. 1] Fig. 1 is a schematic view for illustrating the constitution of a conventional surface plasmon-field enhanced fluorescence spectroscopy apparatus.

[Fig. 2a] Fig. 2a is a schematic view for illustrating one specific example of the sensor chip of the present invention that comprises a single sensor section.

[Fig. 2b] Fig. 2b is a schematic view for illustrating one specific example of the sensor chip of the present invention that comprises one sensor section and has a flow channel formed thereon.

[Fig. 2c] Fig. 2c is a schematic view for illustrating one specific example of the sensor chip of the present invention that comprises three sensor sections. It is noted here that Figs. 2a, 2b and 2c are collectively referred to as "Fig. 2".

[Fig. 3] Fig. 3 is a schematic view for illustrating one specific example of the steps of a blocking treatment performed for the preparation of the sensor chip of the present invention.

[Fig. 4] Fig. 4 is a schematic view that illustrates one specific example of a sensor chip comprising three sensor sections, in which the whole wetted surface between a measurement sample and a metal thin film is divided into regions each containing one of the sensor sections and the entirety of each region is subjected to a blocking treatment with a blocking agent suitable for the region.

MODE FOR CARRYING OUT THE INVENTION

The sensor chip of the present invention will now be described in detail referring to the drawings; however, the sensor chip of the present invention is not restricted to the specific examples shown in the drawings.

1. Constitution of Sensor Chip

The sensor chip of the present invention is:

"a sensor chip for surface plasmon-field enhanced spectroscopy, comprising: a dielectric member: a metal thin film formed on a main surface of the dielectric member; and a region on a part of the metal thin film, where a capturing substance that specifically captures a substance to be measured is immobilized,
wherein a blocking treatment with a blocking agent is performed in a region that includes the region where the capturing substance is immobilized, and the blocking treatment is not performed over the entirety of a wetted surface between a measurement sample and the metal thin film.

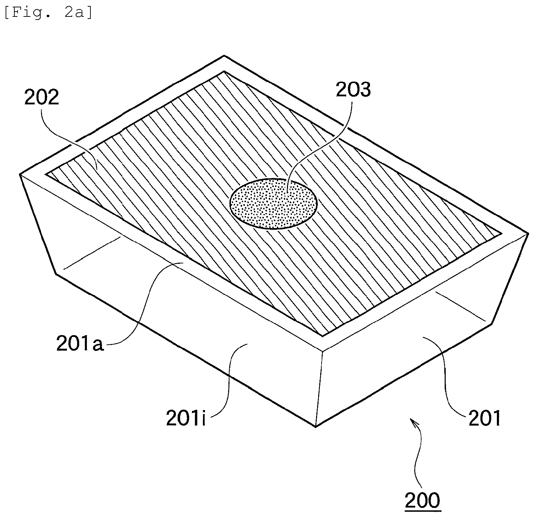

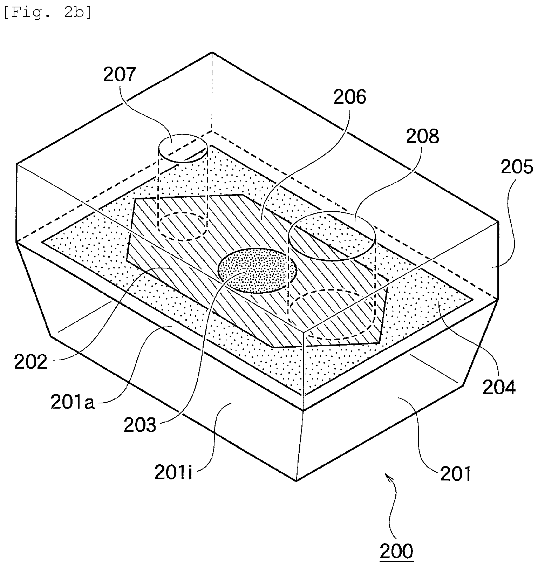

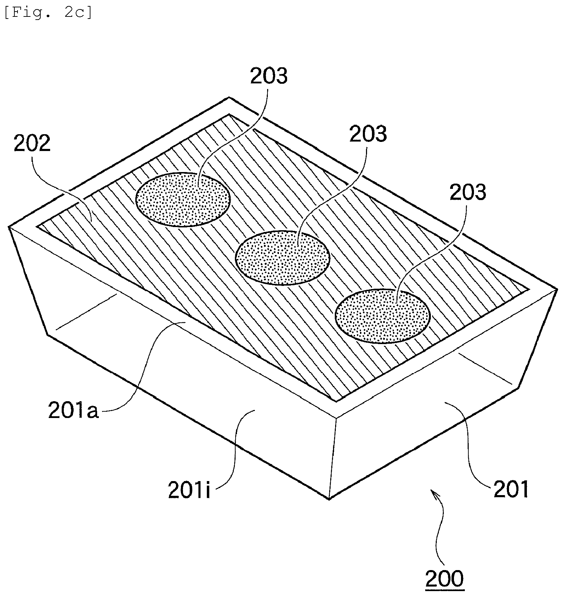

The principal constitution of the sensor chip of the present invention will be described based on one specific example of the sensor chip of the present invention shown in Fig. 2. Fig. 2a shows a specific example of the sensor chip that comprises a single sensor section. It is noted here that members such as a cover are omitted in Fig. 2a. Fig. 2b shows a case where a flow channel is formed using the specific example of the sensor chip that comprises a single sensor section. Fig. 2c shows a specific example of the sensor chip that comprises three sensor sections. It is noted here that, in Fig. 2c as well, members (such as a cover) forming a flow channel are omitted for simplification.

As shown in Figs. 2a and 2c, the specific example of a sensor chip 200 comprises: a dielectric member 201; a metal thin film 202, which is formed on a main surface 201a of the dielectric member 201; and a sensor section 203, which is arranged on a part of the metal thin film 202. The term "sensor section" used herein refers to a region where a capturing substance that specifically captures a substance to be measured is immobilized, and the sensor section is formed on a part of the metal thin film 202. A blocking treatment with a blocking agent is performed in a region that includes the sensor section 203; however, this blocking treatment is not performed over the entirety of a wetted surface between a measurement sample and the metal thin film 202, that is, the entirety of the metal thin film 202 in a flow channel 206. Specifically, this sensor chip is used in a configuration in which a flow channel and the like are formed as exemplified in Fig. 2b.

The sensor chip shown in Fig. 2b has the same constitution as the sensor chips shown in Figs. 2a and 2c, and Fig. 2b shows a specific example of a state where a flow channel and the like are formed. It is note here that, although Fig. 2b is a specific example of the sensor chip that comprises a single sensor section as shown in Fig. 2a, the sensor chip may also take the same constitution when the sensor chip comprises a plurality of sensor sections as shown in Fig. 2c. As shown in Fig. 2b, in the specific example of the sensor chip 200, by arranging a thin-layer member 204 on the metal thin film 202 and placing a plate (cover) 205 on the thin-layer member 204, the flow channel 206 through which a measurement sample flows is formed on the metal thin film 202 comprising the sensor section 203. The measurement sample is introduced via an inlet/outlet 207 to the flow channel 206 using a pipet or the like, and the thus introduced measurement sample is retained in a liquid-retaining section 208.

The sensor chip 200 is used by being mounted on an SPFS apparatus. Upon measurement, the measurement sample is injected via the inlet/outlet 207. By injecting the measurement sample via the inlet/outlet 207 using a pipet or the like and allowing the measurement sample to react for a prescribed time, the substance to be measured is captured by the capturing substance immobilized on the sensor section 203. Subsequently, the measurement sample is discharged via the inlet/outlet 207 using a pipet or the like. Then, after injecting a washing liquid (e.g., PBS in which a surfactant is dissolved) via the inlet/outlet 207 to wash away the measurement sample remaining in the flow channel 206 and the substance to be measured that is non-specifically adsorbing to the flow channel 206, the washing liquid in which the thus washed materials are dissolved is suctioned via the inlet/outlet 207. Next, in order to label the substance to be measured that is captured on the sensor section 203 using a fluorescent substance, a solution of the florescent substance is injected via the inlet/outlet 207, allowed to react for a prescribed time in the same manner as the measurement sample, and then suctioned via the inlet/outlet 207. A washing liquid is again injected via the inlet/outlet 207 to wash away the solution of the florescent substance remaining in the flow channel 206 and the fluorescent substance non-specifically adsorbing to the flow channel 206, and the washing liquid in which the thus washed materials are dissolved is then suctioned via the inlet/outlet 207. Lastly, a measurement liquid (e.g., PBS) is injected via the inlet/outlet 207 and, with the flow channel 206 being filled with this measurement liquid, the sensor section is irradiated with an excitation light and the fluorescence emitted by the thus excited fluorescent substance is measured by surface plasmon-field enhanced fluorescence spectroscopy.

In the present invention, the term "measurement sample" refers to a sample that is subjected to an SPFS apparatus on which the sensor chip of the present invention is mounted, and the measurement sample is, for example, a biological sample, namely a specimen collected from a human or animal subject, or a sample containing a substance originated from a biological sample. Further, in the present invention, the term "substance to be measured" refers to a substance to be detected or quantified using an SPFS apparatus on which the sensor chip of the present invention is mounted, and the substance to be measured is, for example, a protein, a lipid, a saccharide, a nucleic acid or other substance that is to be detected or quantified in the measurement sample.

## 2. Dielectric Member

In the above-described specific examples of the sensor chip of the present invention (Fig. 2), the dielectric member 201 is formed in a hexahedral shape having a trapezoidal cross-section. The upper surface of the dielectric member 201 is the main surface 201a, and one of the sides of this hexahedron is an incident surface 201i, which is the incident surface of an excitation light.

The shape of the dielectric member 201 is not restricted to the above-described hexahedral shape. The dielectric member 201 may take any shape as long as it is configured in such a manner that the dielectric member 201 comprises at least the main surface 201a on which the sensor section 203 is formed and the incident surface 201i through which an excitation light enters; and that the excitation light entering through the incident surface 201i passes through the inside of the dielectric member 201 and irradiates the sensor section 203 at a prescribed incident angle θ that satisfying a total reflection condition. The shape of the dielectric member 201 may be, for example, a conical shape, a pyramidal shape, such as a triangular pyramid shape or a quadrangular pyramid shape, or a semicylindrical shape. Further, two or more incident surfaces 201i may be formed on the dielectric member 201.

The material of the dielectric member is not particularly restricted as long as the dielectric member is made of a material that is optically transparent at least to the excitation light and, from the standpoint of providing an inexpensive sensor chip having excellent ease of handling, it is preferred that the dielectric member be made of, for example, a resin material. For those cases where the dielectric member is made of a resin material, examples of the resin material that can be used include polyesters such as polyethylene terephthalate (PET) and polyethylene naphthalate; polyolefins such as polyethylene (PE) and polypropylene (PP); polycyclic olefins such as cyclic olefin copolymers (COC) and cyclic olefin polymers (COP); vinyl resins such as polyvinyl chloride and polyvinylidene chloride; acrylic resins such as polystyrene, polyether ether ketone (PEEK), polysulfone (PSF), polyether sulfone (PES), polycarbonate (PC), polyamide, polyimide and polymethyl methacrylate resin (PMMA); and triacetylcellulose (TAC).

The method of forming the dielectric member is also not particularly restricted and, for example, when the above-described resin material is used, the dielectric member can be formed by injection molding.

## 3. Metal Thin Film

For the metal thin film 202 (see Fig. 2), the same metal as that of a metal thin film that constitutes a sensor chip used in an ordinary SPFS apparatus can be used. That is, the metal thin film is preferably made of at least one metal selected from the group consisting of gold, silver, aluminum, copper and platinum, among which gold is more preferred. These metals may be in an alloy form or in a laminated form.

As a method of forming the metal thin film on the main surface of the dielectric member, a commonly used method can be employed and, for example, the metal thin film can be formed on the main surface of the dielectric member by a vacuum film-forming method such as electron beam-heating vacuum vapor deposition, resistance heating vacuum vapor deposition, magnetron sputtering, plasma-assisted sputtering, ion-assisted vapor deposition or ion plating.

The thickness of the metal thin film is preferably 5 to 500 nm when the metal thin film is made of gold, silver, aluminum, copper, platinum or an alloy of these metals. From the standpoint of electric field-enhancing effect, the thickness of the metal thin film is more preferably 20 to 70 nm (gold), 20 to 70 nm (silver), 10 to 50 nm (aluminum), 20 to 70 nm (copper), 20 to 70 nm (platinum) or 10 to 70 nm (an alloy of these metals). When the thickness of the metal thin film is in the above-described range, surface plasmon is easily generated, which is preferred.

## 4. Sensor Section

The sensor section 203 (see Fig. 2) is arranged on a partial region of the metal thin film 202, and a capturing substance is immobilized in this region. In this case, a plurality of sensor sections may be arranged, and different capturing substances may be immobilized in the respective sensor sections (see Fig. 2c).

The capturing substance is a substance that specifically captures a substance to be measured (a protein, a lipid, a saccharide, a nucleic acid or other substance). Examples of the capturing substance include antibodies for antigens; enzymes for substrates and coenzymes; receptors for hormones; protein A and protein G for antibodies; avidins for biotin; calmodulin for calcium; and lectin for saccharides. When the substance to be measured is a nucleic acid, a nucleic acid having a sequence that specifically binds thereto can also be used as the capturing substance.

As a method of immobilizing the capturing substance on the metal thin film, a commonly used method can be employed. For example, the capturing substance can be immobilized on the metal thin film by introducing a modifying group that generates a specific bond to the surface of the metal thin film, introducing a reactive group that corresponds to this modifying group to the capturing substance, and then allowing the modifying group and the reactive group to bind with each other.

Specifically, for example, after forming a dielectric layer on the metal thin film as required, the surface of the metal thin film is modified with amino groups by treatment with a silane coupling agent having an amino group at a terminal and the thus modified metal thin film is subsequently treated with NHS (*N*-hydroxysuccinimide) -PEG4-biotin to allow biotin to bind to the amino groups. This biotin is then allowed to react with avidin and a biotinylated capturing substance (e.g., an antibody) is further allowed to react, thereby the capturing substance can be immobilized on the metal thin film. Alternatively, the capturing substance can also be immobilized on the metal thin film by modifying the surface of the metal thin film with carboxyl groups by treatment with a silane coupling agent having a carboxyl group at a terminal, subsequently treating the thus modified metal thin film with EDC (1-ethyl-3-[3-dimethylaminopropyl]carbodiimide hydrochloride) and NHS to perform active esterification of the carboxyl groups, and then allowing an amino group-containing capturing substance (e.g., an antibody) to react with the metal thin film.

Further, as required, a SAM (Self-Assembled Monolayer) may be formed on the surface of the metal thin film to immobilize the capturing substance on the metal thin film. The SAM functions as a foundation in the immobilization of the capturing substance on the metal thin film.

As a monomolecule to be contained in the SAM, for example, a carboxyalkanethiol having about 4 to 20 carbon atoms (available from, for example, Dojindo Laboratories and Sigma-Aldrich Japan), particularly preferably 10-carboxy-1-decanethiol is used. A carboxyalkanethiol having 4 to 20 carbon atoms is suitable because a SAM formed therefrom has small optical influence, that is, the SAM has properties of high transparency, low refractive index, small thickness and the like.

The method of forming such a SAM is not particularly restricted, and a conventionally known method can be employed. Specific examples thereof include a method of immersing the metal thin film in an ethanol solution containing 10-carboxy-1-decanethiol (manufactured by Dojindo Laboratories). In this manner, the thiol group of 10-carboxy-1-decanethiol is bound and immobilized with the metal and self-assembled on the surface of the metal thin film to form a SAM. The method of immobilizing the capturing substance on the thus formed SAM is also not particularly restricted, and a conventionally known method can be employed. For example, a method of treating the SAM with EDC and NHS as described above can be employed.

The shape and area of the region where the capturing substance is immobilized on the metal thin film, that is, the shape and area of the sensor section, are not particularly restricted; however, the area is preferably not smaller than the area of the region irradiated with the incoming excitation light. Particularly, in order to improve the S/N value in the measurement of fluorescence excited in the sensor section, it is preferred that the sensor section have the same shape as the region irradiated with the excitation light. In this case, in order to immobilize the capturing substance only on a partial region of the metal thin film, in accordance with the shape of region where the capturing substance is to be immobilized, for example, a member such as a solution-retaining member 304 may be arranged at a prescribed position on the metal thin film, and the reagent and the like that are used in the above-described method of immobilizing the capturing substance on the metal thin film may then be added to this member.

It is noted here that the sensor section is not restricted to the above-described case where it is formed on the metal thin film of the flow channel, and the sensor section may also be formed on a metal thin film in a well of a welled plate.

5. Blocking Treatment

In the present invention, the term "blocking treatment" refers to a treatment for inhibiting non-specific adsorption or binding of contaminants (proteins, lipids, saccharides and the like that are not the substance to be measured) and a fluorescent labeling substance (s) that are contained in the measurement sample to the sensor section. In the present invention, the term "blocking agent" refers to a substance that inhibits such adsorption or binding.

In the sensor chip 200 of the present invention (see Fig. 2), a blocking treatment with a blocking agent is performed in a region on the metal thin film 202 that includes the region (sensor section 203) where the capturing substance is immobilized . However, this blocking treatment is not performed on the entirety of a wetted surface between the measurement sample and the metal thin film 202.

As described above, according to the investigation by the present inventors, the cause of reduction with time in the effect of blocking autofluorescence emitted by the dielectric member is partial detachment of the metal thin film from the dielectric member, and such detachment of the metal thin film from the dielectric member is influenced by a blocking agent existing on the metal thin film. Accordingly, in order to inhibit the detachment of the metal thin film from the dielectric member, it is effective to perform blocking treatment only in a necessary region that includes the region where the capturing substance is immobilized (sensor section), preferably only in the region where the capturing substance is immobilized (sensor section).

Further, in cases where the sensor chip has plural regions where the capturing substance is immobilized, it is preferred that at least one of the blocking agents used for the blocking treatment performed in each region where the capturing substance is immobilized be different from other blocking agents. In the case of a sensor chip in which different kinds of capturing substances are each immobilized in different regions (for example, in the case of the sensor chip shown in Fig. 2c), in order to realize both inhibition of non-specific adsorption of contaminants and prevention of reduction in the effect of blocking autofluorescence emitted by the dielectric member, it is effective to perform blocking treatment only in the respective sensor sections using blocking agents suitable for the substance captured by the capturing substance immobilized in each sensor section, that is, blocking agents suitable for the substance to be measured that is captured in each sensor section. When all of the sensor section regions are block-treated with the same blocking agent, since the blocking effect varies depending on the substance to be measured that is captured in each sensor section, there are cases where the blocking effect cannot be optimized. Therefore, for example, in the case of the sensor sections 203 shown in Fig. 2c, it is preferred that blocking be performed only in the respective sensor sections using blocking agents suitable for the substance to be measured that is captured in each region, and this enables to achieve a high-sensitivity measurement with reduced noise even in a so-called multi-assay.

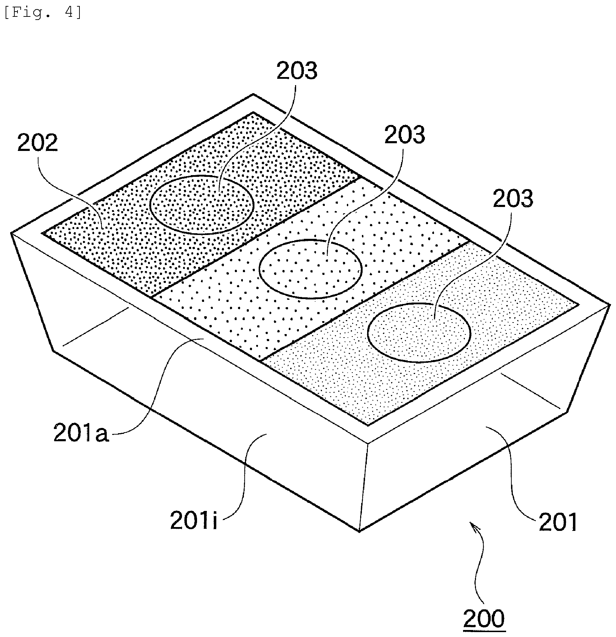

In cases where the sensor chip has plural regions (sensor sections) on the metal thin film where the capturing substance is immobilized, the whole wetted surface between the measurement sample and the metal thin film may be divided into regions each containing one of the sensor sections, and a blocking treatment may be performed on the entirety of each region using a blocking agent suitable for the region, that is, a blocking agent suitable for the substance to be measured that is captured in the sensor section of the region (see, for example, Fig. 4). In this manner, in the sensor chip that has plural sensor sections where different substances to be measured are captured, at least each region of the sensor sections be subjected to a blocking treatment suitable for the respective sensor sections, and it is preferred that the blocking treatment be performed with a different blocking agent for each sensor section. By performing the blocking treatment in this manner, as described above, a high-sensitivity measurement with reduced noise can be achieved even in a multi-assay.

Neither the blocking agent nor the blocking treatment method is particularly restricted, and commonly used blocking agent and blocking treatment method may be employed. Examples of known blocking agents include skim milk, fish gelatin, bovine serum albumin (BSA), surfactants, casein, protamine, polyethylene glycols, trehalose and dextran, and an appropriate blocking agent can be selected in accordance with the measurement sample and the substance to be measured. Thereamong, bovine serum albumin, casein, gelatin and skim milk are more commonly used.

The blocking agent is selected, for example, by the following method. That is, a sensor section-containing region of the sensor chip of the present invention is subjected to a blocking treatment with a candidate blocking agent and, after mounting the thus treated sensor chip on an SPFS apparatus, a measurement sample is introduced to the flow channel of the sensor chip to capture the substance to be measured on the sensor section. Then, the thus captured substance to be measured is labeled with a fluorescent substance, and the sensor section is irradiated with an excitation light to measure fluorescence. Fluorescence is also measured in the same manner for the sensor chip that is not subjected to a blocking treatment and, by comparing the thus measured fluorescence of these sensor chips, a blocking agent that reduces the observed noise (a total of blank signal and baseline signal) to a level that conforms to the intended purpose can be selected.

Examples of a preferred blocking agent for a substance to be measured include: casein as a blocking agent for troponin I; gelatin as a blocking agent for NT-ProBNP; and BSA as a blocking agent for D-dimer.

By further incorporating a saccharide into the blocking agent, the resulting blocking agent is allowed to exhibit the effects of stably protecting the structure of a capturing substance, particularly a protein-capturing substance such as an antibody, and inhibiting a reduction with time in the capturing effect of a substance to be measured. The saccharide is preferably at least one saccharide selected from the group consisting of monosaccharides (e.g., glucose and fructose), disaccharides (e.g., sucrose and maltose) and oligosaccharides constituted by 3 to 10 monosaccharides (e.g., raffinose and panose). Further, the amount of the saccharide(s) contained in a blocking agent solution is preferably 1 to 20% by weight, more preferably 5 to 12% by weight, with respect to the amount of the blocking agent solution.

As a method of the blocking treatment, for example, after adding a blocking agent in a solution state to the surface of the sensor section of the metal thin film in such a manner to cover the sensor section and maintaining this condition for a prescribed time, excess blocking agent solution can be removed from the sensor section of the metal thin film. Specifically, for example, after maintaining the sensor section at room temperature for 1 hour in a state of being covered with a blocking agent solution, the blocking agent solution is removed and the whole sensor chip is dried in such a manner that the surfaces of the sensor section and the like covered with the blocking agent solution are dried.

It is preferred that the blocking treatment be performed only in the region where the capturing substance is immobilized, that is, the sensor section 203. The reason for this is because, by minimizing the blocking treatment with a blocking agent as much as possible, the effect of the blocking agent (e.g., residual moisture) on the detachment of the metal thin film is minimized.

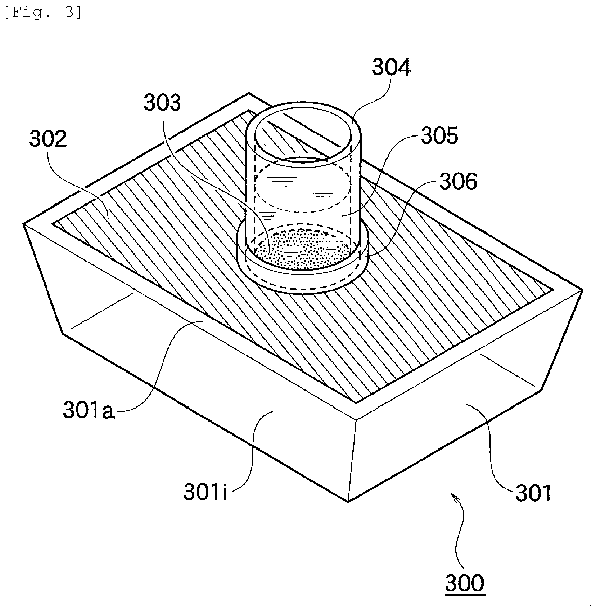

As an example of a method of blocking only the region (sensor section) on the metal thin film where the capturing substance is immobilized, the steps shown in Fig. 3 will now be described. When a sensor section 303 is circular, a cylindrical solution-retaining member 304, which has an inner diameter of the same dimension as the sensor section, is arranged on the sensor section 303 of a metal thin film 302 such that the sensor section 303 is configured inside the inner diameter of the blocking solution-retaining member. The material of the solution-retaining member 304 is not particularly restricted as long as a blocking agent solution can be retained therein, and examples thereof include resins such as polystyrenes (PS), polypropylenes (PP) and polymethyl methacrylate resins (PMMA). Next, a blocking agent solution 305 is added to the solution-retaining member 304 using a pipet or the like such that the entirety of the sensor section 303 is covered with the blocking agent solution, and this condition is maintained for a prescribed time, for example, 1 hour. In this case, in order to prevent the blocking agent solution 305 placed in the solution-retaining member 304 from leaking through a gap between the solution retaining member 304 and the metal thin film 302, the gap between the solution-retaining member 304 and the metal thin film 302 is sealed. This sealing is provided by, for example, a seal member 306 which is wrapped around the bottom part of the solution-retaining member 304. The material of the seal member 306 is, for example, a rubber, and in order to allow the seal member 306 to exhibit its effect more sufficiently, it is preferred that the seal member 306 be tightly adhered to the surface of the metal thin film 303 with application of a pressure from above the solution-retaining member 304 by, for example, a method of sandwiching the blocking agent solution-retaining member 304 and a dielectric member 301 with appropriate plate members (not shown) and applying thereto a pressure. After maintaining this condition at room temperature for a prescribed time (for example, 1 hour), the blocking agent solution 305 is removed from the solution-retaining member 304 using a pipet or the like, and the resulting sensor chip is dried in an incubator.

The blocking treatment on the sensor section 303 may be performed using a plurality of blocking agents. For example, depending on the measurement sample and the substance to be measured, when a high blocking effect is attained by the use of a plurality of blocking agents, by treating the sensor section 303 with a certain kind of blocking agent solution and then with another kind of blocking agent solution, the sensor section 303 can be coated with a layer made of the two kinds of blocking agents.

As described above, the shape of the sensor section is not restricted to a circular shape and, when the sensor section takes a non-circular shape, a blocking agent solution-retaining member that conforms to this shape can be arranged. Further, for example, when the sensor chip has plural sensor sections as shown in Fig. 2c, the above-described method can be performed on the plural sensor sections.

As described above, when the sensor chip has plural regions (sensor sections) on the metal thin film where the capturing substance is immobilized, the whole wetted surface between the measurement sample and the metal thin film may be divided into regions each containing one of the sensor sections, and a blocking treatment may be performed on the entirety of each region using a blocking agent suitable for the region (see, for example, Fig. 4). In this case, the regions can be separated from one another using an appropriate member such that the blocking agent of one region does not come into contact with other region, and a blocking treatment can be performed on each region using a blocking agent suitable for the region.

6. Detection and Quantification of Substance to be Measured in Measurement Sample

The sensor chip of the present invention can be mounted on an SPFS apparatus and used for the detection and quantification of a substance to be measured that is contained in a measurement sample. As the SPFS apparatus, a conventionally known SPFS apparatus can be employed. For example, capturing of the substance to be measured to the sensor section, fluorescent labeling of the thus captured substance to be measured (e.g., the use of a labeling secondary antibody), irradiation of an excitation light, and measurement of resonance between evanescent wave and surface plasmon generated by the metal thin film and the thus emitted fluorescence are also not particularly restricted and can be performed by a variety of conventionally known methods .

As described above, one specific example of the sensor chip of the present invention is a sensor chip in which, as shown in Fig. 2b, the flow channel 206 through which a measurement sample flows is formed on the metal thin film 202 containing the sensor section 203 by arranging the thin-layer member 204 on the metal thin film 202 and placing the plate (cover) 205 over the thin-layer member 204. In this case, the material of the thin-layer member 204 is, for example, an acrylic adhesive sheet, and the thickness thereof may be decided in accordance with the desired height of the flow channel and is, for example, 20 to 1,000 μm. The material of the plate (cover) 205 is, for example, the same resin material as that of the above-described dielectric member. The shapes of the inlet/outlet 207 and the liquid-retaining section 208 can be set as appropriate such that the measurement sample retained in the liquid-retaining section 208 is easily stirred by, as described above, introducing the measurement sample via the inlet/outlet 207 using a pipet or the like and repeatedly suctioning and injecting the measurement sample several times via the inlet/outlet 207 using a pipet or the like.

Further, as described above, the sensor chip of the present invention is not restricted to a mode where it is used in a configuration in which the above-described flow channel is formed, and the sensor chip of the present invention may also be used in other mode, for example, a mode where the sensor chip whose sensor section is formed in a well of a welled plate is mounted on an SPFS apparatus.

As described above, the measurement sample is, for example, a biological sample (a specimen collected from a human or animal subject); however, for the actual measurement, the measurement sample is preferably a liquid specimen. Representative examples of such a measurement sample include blood (including serum and plasma) and urine. When cells are the subject, a suspension of the cells can be prepared in accordance with a prescribed method to obtain a liquid measurement sample. Further, as required, a collected specimen may be subjected to an anticoagulation treatment, centrifugation, extraction and/or other necessary treatments before being used as a measurement sample.

The substance to be measured is, as described above, a protein, a lipid, a saccharide, a nucleic acid or other substance that is to be detected or quantified in a measurement sample. When the measurement sample is blood, examples of the substance to be measured include myocardial markers such as troponin I, NT-ProBNP and D-dimer.

EXAMPLES

The present invention will now be described in more detail by way of examples thereof; however, the present invention is not restricted thereto.

[Example 1]

(1) Preparation of Sensor Chip

Step (a) : First, on the main surface of a prism member having a substantially trapezoidal cross-section which was prepared using a cycloolefin polymer resin ZEONEX (registered trademark, manufactured by ZEON Corporation) as a material of a dielectric member, a chromium thin film was formed by sputtering, and a gold thin film was further formed on the surface of the chromium thin film by sputtering. The thickness of the chromium thin film was 1 to 3 nm and that of the gold thin film was 44 to 52 nm.

Step (b): The resulting prism on which these films were formed was immersed in an ethanol solution containing 1 mM of 10-carboxy-1-decanethiol for at least 24 hours to form a SAM (Self-Assembled Monolayer) on one side of the gold thin film. The prism was then removed from the solution and washed with ethanol and isopropanol, followed by drying using an air gun.

Step (c): On one end of a cylindrical member that was made of a polymethyl methacrylate resin (PMMA) and had an outer diameter of 7 mm, an inner diameter of 5 mm and a length of 15 mm, a groove of 0.5 mm in depth and 1 mm in width was formed, and a solution-retaining member fitted with a fluororubber O-ring of 1mm in thickness and 6 mm in inner diameter (manufactured by Misumi Corporation) was placed on the metal thin film of the prism obtained in the step (b). In order to inhibit leakage from the solution-retaining member, the solution-retaining member and the prism were sandwiched by two stainless-steel plates from the top and bottom and then screw-fixed (on the upper stainless-steel plate, an opening through which reagents and the like were supplied to and removed from the solution-retaining member was arranged) .

Step (d): After introducing 0.2 mL of MES [2-morpholinoethanesulfonic acid]-buffered physiological saline (pH 6.0) containing 25 mg/mL of N-hydroxysuccinimide [NHS] and 25 mg/mL of water-soluble carbodiimide [EDC] and allowing it to react for 20 minutes, the resulting reaction liquid was withdrawn, and 0.2 mL of an acetate solution (pH 6.0) containing an anti-troponin I [TnI] monoclonal antibody was further introduced and allowed to react for 30 minutes, thereby immobilizing a primary antibody on the SAM.

Step (e) : Next, 0.2 mL of 50 mM Tris (pH 7.4) was introduced and allowed to react for 15 minutes so as to deactivate unreacted active ester groups.

Step (f): As a blocking agent solution, a PBS-buffered physiological saline containing 1% by weight of casein and 10% by weight of sucrose was introduced and left to stand for 30 minutes . Then, this blocking agent solution was withdrawn, and a non-specific adsorption-inhibiting treatment was performed on the region where the primary antibody was immobilized.

Step (g): The solution-retaining member was removed and, using a PET substrate double-sided tape No. 5610 (manufactured by Nitto Denko Corporation) on which a through-hole (7 mm × 30 mm) for the formation of a flow channel was formed (thin-layer member 204), a 10 mm-thick polymethyl methacrylate plate having an inlet/outlet 207 and a liquid-retaining section 208 was adhered to form a flow channel 206, thereby preparing a sensor chip.

(2) Comparison of Sensor Chip between Immediately After Preparation and After Storage

(2-1) Measurement using Sensor Chip Immediately After Preparation

Antigen Addition Step: A PBS-buffered physiological saline containing 0.1 ng/mL of troponin I as an antigen and 1% by weight of bovine serum albumin [BSA] was injected into the sensor chip prepared in the above (1), and the immobilized primary antibody and the antigen were allowed to react for 30 minutes. The resulting antigen-containing solution was suction-removed, and the sensor chip was washed by repeating several times the operation of injecting and suction-removing a TBS containing 0.05% by weight of Tween 20.

Measurement of baseline signal: Here, using a laser light source, the metal thin films of the sensor chip were irradiated with a laser light having a wavelength of 635 nm whose photon amount was adjusted by an optical filter (manufactured by Sigmakoki Co., Ltd.), and the blank fluorescence (baseline signal) was detected using a CCD image sensor (manufactured by Texas Instruments Inc.) equipped with a cut filter for cutting light having wavelengths of non-fluorescent components and an objective lens ($\times$20).

Labeled Antibody Addition Step: The TBS containing 0.05% by weight of Tween 20 was suction-removed, and a PBS-buffered physiological saline that contained an anti-troponin I [TnI] monoclonal antibody (a clone different from the primary antibody) fluorescently labeled using Alexa Fluor (registered trademark) 647 Protein Labeling Kit (manufactured by Invitrogen Corp.) was injected into the flow channel and allowed to react for 15 minutes, thereby forming an immunocomplex. The PBS-buffered physiological saline, a solution containing the fluorescently labeled antibody, was suction-removed, and the sensor chip was subsequently washed by repeating several times the operation of injecting and suction-removing a TBS containing 0.05% by weight of Tween 20.

Measurement of assay signal: The fluorescence signal attributed to the immunocomplex was measured by detecting fluorescence in the same manner as in the measurement of the baseline signal.

Measurement of blank signal: The measurement of blank signal attributed to non-specific adsorption of the labeled antibody was performed in the same manner as described above except that, in the antigen addition step, a PBS-buffered physiological saline containing 1% by weight bovine serum albumin [BSA] without troponin I as an antigen was injected into other sensor chip.

Calculation of S/N: From the thus obtained signals of the noise components (baseline signal and blank signal) and the signals obtained using the antigen-containing solution, the S/N value was calculated using the following formula:

$$\text{S/N} = |(\text{Assay signal})|/|(\text{Baseline signal} + \text{Blank signal})|$$

Evaluation of Defect in Metal Thin Films: The presence or absence of a defect in the metal thin films was verified by observing the surface conditions of the metal thin films under a light microscope. The results thereof are shown in Table 1.

(2-2) Measurement using Sensor Chip after Storage

After storing the sensor chip prepared by the method of (1) at 4°C for 60 days, the baseline signal and the assay signal were measured in the same manner as in (2-1) to calculate the S/N value. Further, the presence or absence of a defect in the metal thin films was also verified in the same manner as in (2-1) by observing the surface conditions of the metal thin films under a light microscope. The results thereof are shown in Table 1.

[Comparative Example 1]

(1) Preparation of Sensor Chip

Steps (a) to (e): A primary antibody was immobilized on a gold thin film formed on a prism in the same manner as in Example 1.

Step (f): The solution-retaining member was removed and, using a PET substrate double-sided tape No. 5610 (manufactured by Nitto Denko Corporation) on which a through-hole (7 mm $\times$ 30 mm) for the formation of a flow channel was formed (thin-layer member 204), a 10 mm-thick polymethyl methacrylate plate having an inlet/outlet 207 and a liquid-retaining section 208 was adhered to form a flow channel 206.

Step (g): Lastly, as a blocking agent solution, a PBS-buffered physiological saline containing 1% by weight of casein and 10% by weight of sucrose was introduced to the flow channel 206 and left to stand for 30 minutes. Then, this blocking agent solution was withdrawn and a non-specific adsorption-inhibiting treatment was performed, thereby preparing a sensor chip.

(2) Comparison of Sensor Chip between Immediately After Preparation and After Storage

(2-1) Measurement using Sensor Chip Immediately After Preparation

In the same manner as in (2-1) of Example 1, the baseline signal and the assay signal were measured, the S/N value was calculated and the presence or absence of a defect in the metal thin films was verified. The results thereof are shown in Table 1.

(2-2) Measurement using Sensor Chip after Storage

In the same manner as in (2-2) of Example 1, after storing the sensor chip prepared by the method of (1), the baseline signal and the assay signal were measured, the S/N value was calculated and the presence or absence of a defect in the metal thin films was verified. The results thereof are shown in Table 1.

[Table 1]

| | Storage period (days) | Noise components | | S/N[*2] | Defect in Metal Films |
| --- | --- | --- | --- | --- | --- |
| | | Baseline signal[*1] | Blank signal[*1] | | |
| Example 1 | 0 | 100% | 100% | 1 | absent |
| | 60 | 100% | 100% | 1 | absent |
| Comparative Example 1 | 0 | 100% | 100% | 1 | absent |
| | 60 | 150% | 100% | 0.8 | present |

[*1]: For each baseline signal and blank signal measured in Example 1 and Comparative Example 1, the value obtained using the sensor chip stored for 60 days is indicated as a value converted into %, taking the value obtained using the sensor chip with no storage period as 100%.
[*2]: Taking the S/N value of the case where the sensor chip was not stored in Comparative Example 1 as 1, the S/N values of other cases are indicated as converted values.

[Example 2]

(1) Preparation of Sensor Chip

Step (a): A prism having a gold film formed thereon was prepared in the same manner as in the step (a) of Example 1.
Step (b): A SAM (Self-Assembled Monolayer) was formed on one side of the gold thin film in the same manner as in Example 1.
Step (c): The same solution-retaining member made of a polymethyl methacrylate resin (PMMA) as the one used in Example 1 was arranged at 3 spots (sensor sections of regions 1 to 3) on the above-prepared prism having a gold film and, in order to inhibit leakage from the solution-retaining member, the solution-retaining member and the prism were sandwiched by two stainless-steel plates from the top and bottom and then screw-fixed (on the upper stainless-steel plate, an opening through which reagents and the like were supplied to and removed from the solution-retaining member was arranged).
Step (d): Next, 0.2 mL of MES [2-morpholinoethanesulfonic acid]-buffered physiological saline (pH 6.0) containing 25 mg/mL of N-hydroxysuccinimide [NHS] and 25 mg/mL of water-soluble carbodiimide [EDC] was introduced to all of the solution-retaining members and allowed to react for 20 minutes, and the resulting reaction liquid was withdrawn. Then, 0.2 mL of an acetate solution (pH 6.0) containing an anti-troponin I [TnI] monoclonal antibody, 0.2 mL of an acetate solution (pH 6.0) containing an anti-NT-ProBNP monoclonal antibody and 0.2 mL of an acetate solution (pH 6.0) containing an anti-D-dimer monoclonal antibody were introduced to each solution-retaining member and allowed to react for 30 minutes, thereby immobilizing different primary antibodies on the SAM.
Step (e): Next, 0.2 mL of 50 mM Tris (pH 7.4) was introduced to each solution-retaining member and allowed to react for 15 minutes so as to deactivate unreacted active ester groups.
Step (f): As a blocking agent solution, a PBS-buffered physiological saline containing 1% by weight of casein and 10% by weight of sucrose was introduced to the region where the anti-troponin I [TnI] monoclonal antibody was immobilized and left to stand for 30 minutes. Then, this blocking agent solution was withdrawn, and a non-specific adsorption-inhibiting treatment was performed on the region where the anti-troponin I [TnI] monoclonal antibody was immobilized.

Step (g): In the same manner, a PBS-buffered physiological saline containing 1% by weight of gelatin and 10% by weight of sucrose was introduced to the region where the anti-NT-ProBNP monoclonal antibody was immobilized and left to stand for 30 minutes. Then, this blocking agent solution was withdrawn, and a non-specific adsorption-inhibiting treatment was performed on the region where the anti-NT-ProBNP monoclonal antibody was immobilized.

Step (h): In the same manner, as a blocking agent solution, a PBS-buffered physiological saline containing 1% by weight of gelatin and 10% by weight of sucrose was introduced to the region where the anti-D-dimer monoclonal antibody was immobilized and left to stand for 30 minutes. Then, this blocking agent solution was withdrawn, and a non-specific adsorption-inhibiting treatment was performed on the region where the anti-D-dimer monoclonal antibody was immobilized.

Step (i): The solution-retaining members were removed and, using a PET substrate double-sided tape No. 5610 (manufactured by Nitto Denko Corporation) on which a through-hole (7 mm × 30 mm) for the formation of a flow channel was formed (thin-layer member 204), a 10 mm-thick polymethyl methacrylate plate having an inlet/outlet 207 and a liquid-retaining section 208 was adhered to form a flow channel 206, thereby preparing a sensor chip having three antibody-immobilized regions (sensor sections of regions 1 to 3).

(2) Comparison of Sensor Chip between Immediately After Preparation and After Storage

(2-1) Measurement using Sensor Chip Immediately After Preparation

Antigen Addition Step: Addition of antigens was performed in the manner as in Example 1, except that a PBS-buffered physiological saline containing 0.1 ng/mL of troponin I, 0.1 ng/mL of NT-ProBNP, 0.1 ng/mL of D-dimer and 1% by weight of bovine serum albumin [BSA] was used as an antigen solution.

Measurement of baseline signal: The baseline signal was measured in the same manner as in Example 1, except that the three antibody-immobilized regions on the sensor chip were each irradiated with the excitation light emitted from the laser light source.

Labeled Antibody Addition Step: This step was performed in the same manner as in Example 1, except that a PBS-buffered physiological saline that contained an anti-troponin I [TnI] monoclonal antibody (a clone different from the primary antibody), an anti-NT-ProBNP monoclonal antibody (a clone different from the primary antibody) and an anti-D-dimer monoclonal antibody (a clone different from the primary antibody), which antibodies were fluorescently labeled using Alexa Fluor (registered trademark) 647 Protein Labeling Kit (manufactured by Invitrogen Corp.), was used as a labeled antibody solution.

Measurement of assay signal: In the same manner as in the measurement of the baseline signal, the three antibody-immobilized regions on the sensor chip were each irradiated with the excitation light, and the fluorescence signal attributed to an immunocomplex was measured in each antibody-immobilized region.

Measurement of blank signal: The measurement of blank signal attributed to non-specific adsorption of each labeled antibody was performed in the same manner as described above except that, in the antigen addition step, a PBS-buffered physiological saline containing 1% by weight bovine serum albumin [BSA] without any antigen was injected into other sensor chip.

Calculation of S/N: From the thus obtained signals of the noise components (baseline signal and blank signal) and the signals obtained using the antigen-containing solution, the S/N value was calculated using the following formula:

```
S/N = |(Assay signal)|/|(Baseline signal + Blank signal)|
```

Evaluation of Defect in Metal Thin Films: The presence or absence of a defect in the metal thin films was verified by observing the surface conditions of the metal thin films under a light microscope. The results thereof are shown in Table 2.

(2-2) Measurement using Sensor Chip after Storage

After storing the sensor chip prepared by the method of (1) at 4°C for 60 days, the baseline signal and the assay signal were measured in the same manner as in (2-1) to calculate the S/N value. Further, the presence or absence of a defect in the metal thin films was also verified in the same manner as in (2-1) by observing the surface conditions of the metal thin films under a light microscope. The results thereof are shown in Table 2.

[Comparative Example 2]

(1) Preparation of Sensor Chip

Steps (a) to (e): The respective primary antibodies were immobilized on a gold thin film formed on a prism in the same manner as in Example 2.

Step (f) : The solution-retaining members were removed and, using a PET substrate double-sided tape No. 5610 (manufactured by Nitto Denko Corporation) on which a through-hole (7 mm $\times$ 30 mm) for the formation of a flow channel was formed (thin-layer member 204), a 10 mm-thick polymethyl methacrylate plate having an inlet/outlet 207 and a liquid-retaining section 208 was adhered to form a flow channel 206.

Step (g): Lastly, as a blocking agent solution, a PBS-buffered physiological saline containing 1% by weight of casein and 10% by weight of sucrose was introduced to the flow channel 206 and left to stand for 30 minutes. Then, this blocking agent solution was withdrawn and a non-specific adsorption-inhibiting treatment was performed, thereby preparing a sensor chip having three antibody-immobilized regions (sensor sections of regions 1 to 3).

(2) Comparison of Sensor Chip between Immediately After Preparation and After Storage

(2-1) Measurement using Sensor Chip Immediately After Preparation

In the same manner as in (2-1) of Example 2, the baseline signals and the assay signals were measured, the S/N values were calculated and the presence or absence of a defect in the metal thin films was verified. The results thereof are shown in Table 2.

(2-2) Measurement using Sensor Chip after Storage

In the same manner as in (2-2) of Example 2, after storing the sensor chip prepared by the method of (1), the baseline signals and the assay signals were measured, the S/N values were calculated and the presence or absence of a defect in the metal thin films was verified. The results thereof are shown in Table 2.

As seen from Table 2, according to Example 2, a sensor chip comprising regions where different kinds of capturing substances are each immobilized, in which sensor chip not only non-specific adsorption of contaminants originating from a measurement sample (biological sample) to the regions (sensor sections) where the respective capturing substances are immobilized is inhibited but also the effect of blocking autofluorescence emitted by the dielectric member is not reduced even with time, can be obtained.

[Table 2]

| Sensor section | | Item (substance to be measured) | Blocking agent | Blocking method | Storage period (days) | Noise components | | S/N[*3] | Defect in Metal Films |
|---|---|---|---|---|---|---|---|---|---|
| | | | | | | Baseline signal[*1] | Blank signal | | |
| Region 1 | Example 2 | cTnI | casein | Introduced to the liquid-retaining section | 0 | 100% | 100% | 1 | absent |
| | | cTnI | casein | Introduced to the liquid-retaining section | 60 | 100% | 100% | 1 | absent |
| | Comparative Example 2 | cTnI | casein | Introduced to the flow channel | 0 | 100% | 100% | 1 | absent |
| | | cTnI | casein | Introduced to the flow channel | 60 | 150% | 100% | 0.8 | present |
| Region 2 | Example 2 | NT-ProBNP | gelatin | Introduced to the liquid-retaining section | 0 | 100% | 10% | 1.82 | absent |
| | | NT-ProBNP | gelatin | Introduced to the liquid-retaining section | 60 | 100% | 10% | 1.82 | absent |
| | Comparative Example 2 | NT-ProBNP | casein | Introduced to the flow channel | 0 | 100% | 100% | 1 | absent |
| | | NT-ProBNP | casein | Introduced to the flow channel | 60 | 150% | 100% | 0.8 | present |

| Sensor section | | Item (substance to be measured) | Blocking agent | Blocking method | Storage period (days) | Noise components | | S/N[*3] | Defect in Metal Films |
|---|---|---|---|---|---|---|---|---|---|
| | | | | | | Baseline signal[*1] | Blank signal | | |
| Region 3 | Example 2 | D-dimer | BSA | Introduced to the liquid-retaining section | 0 | 100% | 91% | 1.05 | absent |
| | | D-dimer | BSA | Introduced to the liquid-retaining section | 60 | 100% | 91% | 1.05 | absent |
| | Comparative Example 2 | D-dimer | casein | Introduced to the flow channel | 0 | 100% | 100% | 1 | absent |
| | | D-dimer | casein | Introduced to the flow channel | 60 | 150% | 100% | 0.8 | present |

[*1]: The baseline signals (%) measured for each region in Example 2 and Comparative Example 2 represent the effect of the storage period in each region in Example 2 and Comparative Example 2. For each region in Example 2 and Comparative Example 2, the baseline signal measured using the sensor chip stored for 60 days is indicated as a value converted into %, taking the value obtained using the sensor chip with no storage period as 100%.

[*2]: The blank signals (%) measured for each region in Example 2 and Comparative Example 2 represent the effect of a difference in the blocking agent in those cases where the same item (substance to be measured) and the same storage period were used. Taking the value obtained in Comparative Example 2 (where casein was used as the blocking agent) for each region with each storage period as 100%, the blank signal measured in Example 2 (where the blocking agent was one suitable for the substance to be measured in each region) is indicated as a value converted into %.

[*3]: For each region, taking the S/N value of the case where the sensor chip was not stored in Comparative Example 2 as 1, the S/N values of other cases were indicated as converted values.

DESCRIPTION OF SYMBOLS

| | |
|---|---|
| 100: | SPFS apparatus |
| 110: | Sensor chip |
| 111: | Sensor chip mounting section |
| 112: | Dielectric member |
| 112a: | Main surface of dielectric member |
| 112i: | Incident surface of dielectric member |
| 113: | Metal thin film |
| 114: | Thin-layer member |
| 115: | Plate (cover) |
| 116: | Sensor section |
| 117: | Fine flow channel |
| 120: | Light source |
| 121: | Excitation light |
| 122: | Reflected light |
| 123: | Light-receiving means |
| 130: | Light-detecting means |
| 131: | Fluorescence |
| 132: | Light-condensing member |
| 133: | Wavelength-selecting function member |
| θ: | Incident angle |
| 200: | Sensor chip |
| 201: | Dielectric member |
| 201a: | Main surface of dielectric member |
| 201i: | Incident plane of dielectric member |
| 202: | Metal thin film |
| 203: | Sensor section |
| 204: | Thin-layer member |
| 205: | Plate (cover) |
| 206: | Flow channel |
| 207: | Inlet/outlet |
| 208: | Liquid-retaining section |
| 300: | Sensor chip |
| 301: | Dielectric member |
| 301a: | Main surface of dielectric member |
| 301i: | Incident plane of dielectric member |
| 302: | Metal thin film |
| 303: | Sensor section |
| 304: | Solution-retaining member |
| 305: | Blocking agent solution |
| 306: | Seal member |

**Claims**

1.  A sensor chip (110, 200, 300) for surface plasmon-field enhanced spectroscopy, comprising:

    a dielectric member (112, 201, 301);
    a metal thin film (113, 202, 302) formed on a main surface of said dielectric member (112, 201, 301); and
    a region (116, 203, 303) on a part of said metal thin film (113, 202, 302), where a capturing substance that specifically captures a substance to be measured is immobilized,
    wherein
    a blocking treatment with a blocking agent is performed in a region that includes said region (116, 203, 303) where said capturing substance is immobilized, and
    said blocking treatment is not performed over the entirety of a wetted surface between a measurement sample and said metal thin film (113, 202, 302),

wherein

said sensor chip (110, 200, 300) comprises plural (116, 203, 303) regions where different kinds of capturing substances are respectively immobilized in the respective plural regions, **characterized in that**

at least one blocking agent used for said blocking treatment performed in one of said plural regions is different from the other blocking agent or agents used for said blocking treatment performed in the other region or regions.

2. The sensor chip (110, 200, 300) according to claim 1, wherein said blocking treatment is performed only in said region (116, 203, 303) where said capturing substance is immobilized.

3. The sensor chip (110, 200, 300) according to any one of claims 1 to 2, wherein said blocking agent contains a substance having a blocking effect and a saccharide.

4. The sensor chip (110, 200, 300) according to any one of claims 1 to 3, wherein said blocking agent is selected from the group comprising bovine serum albumin, casein, gelatin and skim milk.

**Patentansprüche**

1. Sensorchip (110, 200, 300) zur Oberflächenplasmonenfeld-verstärkten Spektroskopie, mit:

einem dielektrischen Element (112, 201, 301);

einem auf einer Hauptfläche des dielektrischen Elementes (112, 201, 301) gebildeten Metall-Dünnfilm (113, 202, 302); und

einem Bereich (116, 203, 303) auf einem Teil des Metall-Dünnfilms (113, 202, 302), in dem ein Fangstoff, der spezifisch eine zu messende Substanz einfängt, immobilisiert ist,

wobei

eine Blockierungsbehandlung mit einem Blockierungsmittel in einem Bereich durchgeführt wird, der den Bereich (116, 203, 303) umfasst, in welchem der Fangstoff immobilisiert ist, und

die Blockierungsbehandlung über die Gesamtheit einer angefeuchteten Fläche zwischen einer Messprobe und dem Metall-Dünnfilm (113, 202, 302) nicht durchgeführt wird,

wobei der Sensorchip (110, 200, 300) mehrere Bereiche (116, 203, 303) aufweist, in denen verschiedene Arten von Fangstoffen jeweils in den jeweiligen mehreren Bereichen immobilisiert sind,

**dadurch gekennzeichnet, dass**

zumindest ein Blockierungsmittel, das verwendet wird für die in einem der mehreren Bereiche durchgeführte Blockierungsbehandlung, sich unterscheidet von dem/den anderen Blockierungsmittel(n), die für die in dem anderen Bereich oder den anderen Bereichen durchgeführten Blockierungsbehandlung verwendet wird/werden.

2. Sensorchip (110, 200, 300) gemäß Anspruch 1, wobei die Blockierungsbehandlung nur in dem Bereich (116, 203, 303) durchgeführt wird, wo der Fangstoff immobilisiert ist.

3. Sensorchip (110, 200, 300) gemäß irgendeinem der Ansprüche 1 bis 2, wobei das Blockierungsmittel eine Substanz, die eine Blockierungswirkung hat, und ein Saccharid enthält.

4. Sensorchip (110, 200, 300) gemäß irgendeinem der Ansprüche 1 bis 3, wobei das Blockierungsmittel ausgewählt ist aus der Gruppe, die Rinderserumalbumin, Kasein, Gelatine und entrahmte Milch umfasst.

**Revendications**

1. Puce de capteur (110, 200, 300) pour la spectroscopie renforcée par champ de plasmons de surface, comprenant :

un membre diélectrique (112, 201, 301) ;

un film métallique mince (113, 202, 302) formé sur une surface principale dudit membre diélectrique (112, 201, 301) ; et

une région (116, 203, 303) sur une partie dudit film métallique mince (113, 202, 302), où une substance capturant, qui capture particulièrement une substance à mesurer est immobilisée ,

dans lequel

un traitement de blocage avec un agent de blocage est effectué dans une région, qui comprend ladite région (116, 203, 303, où ladite substance capturant est immobilisée, et

ledit traitement de blocage n'est pas effectué sur la totalité d'une surface mouillée entre un échantillon de mesure et ledit film métallique mince (113, 202, 302),

dans lequel

ladite puce de capteur (110, 200, 300) comprend plusieurs régions (116, 203, 303) où de différents types de substances capturant sont chacune immobilisées dans les plusieurs régions respectives,

**caractérisé en ce que**

au moins un agent de blocage utilisé pour ledit traitement de blocage effectué dans une des plusieurs régions est différent des autres agents de blocage ou de l'autre agent de blocage utilisé(s) pour ledit traitement de blocage effectué dans l'autre région ou dans les autres régions.

2. Puce de capteur (110, 200, 300) selon la revendication 1, dans lequel ledit traitement de blocage n'est effectué que dans ladite région (116, 203, 303) où ladite substance capturant est immobilisée.

3. Puce de capteur (110, 200, 300) selon l'une quelconque des revendications 1 à 2, dans lequel ledit agent de blocage contient une substance ayant un effet de blocage et un saccharide.

4. Puce de capteur (110, 200, 300) selon l'une quelconque des revendications 1 à 3, dans lequel ledit agent de blocage est sélectionné dans le groupe comprenant de l'albumine de sérum bovin, de la caséine, de la gélatine et du lait écrémé.

[Fig. 1]

[Fig. 2a]

[Fig. 2b]

[Fig. 2c]

[Fig. 3]

[Fig. 4]

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2009079978 A
- JP 2006292472 A